# EUROPEAN PATENT SPECIFICATION

(11) **EP 1 992 705 B1**
(45) Date of publication and mention of the grant of the patent: **11.01.2012**
(21) Application number: 08008920.4
(22) Date of filing: 14.05.2008
(51) Int. Cl.: C12Q 1/68

(54) **DETECTING INTERACTIONS USING APTAMERS**
ERKENNUNG VON INTERAKTIONEN UNTER VERWENDUNG VON APTAMEREN
DETECTION D`INTERACTIONS A L`AIDE D`APTAMERS

(30) Priority: 14.05.2007 JP 2007128188
(43) Date of publication of application: 19.11.2008
(73) Proprietor: Sony Corporation, Tokyo (JP)
(72) Inventor: Watanabe, Yuuki, Tokyo (JP); Ueno, Masatsugu, Tokyo (JP)
(74) Representative: Müller - Hoffmann & Partner

(56) References cited:
- EP-A- 1 521 077
- WO-A-00/22167
- US-A1- 2003 162 211
- US-A1- 2006 040 276
- US-B1- 6 261 783

## Description

### CROSS REFERENCES TO RELATED APPLICATIONS

The present invention contains subject matter related to Japanese Patent Application JP 2007-128188 filed in the Japan Patent Office on May 14, 2007.

### BACKGROUND OF THE INVENTION

### 1. Field of the Invention

This invention relates to detecting nucleic acid strands and detection method usable for the detection of specific substances or bindings or interactions between substances. More specifically, the present invention is concerned with detecting nucleic acid strands capable of detecting specific substances or bindings or interactions between substances and also with a detection method for bindings or interactions between substances.

### 2. Description of the Related Art

Technologies that detect proteins, organic small molecules, nucleic acids, multimeric complexes, cells, cell tissues, metal ions, microorganisms, viruses and the like bear an extremely important part in research, developments and the like in a wide variety of fields. Developments of detection technologies are now under way in all areas such as, for example, the diagnosis of specific diseases, drug developments, hygienic control of foods and the like, and cleanup and restoration of environmental pollutions.

Among these, advanced especially in recent years are technologies that allow an interaction or reaction to proceed between substance on a surface (interface) of a substrate or the like and detect it by a physical means, optical means or the like. These technologies have already found increasing utility as critical technologies in areas such as the diagnosis of diseases, screening of compounds such as drugs, forensic medicine, comprehensive analysis of genetic information, function analysis of biosubstances, proteome analysis, and analysis of *in vivo* interactions.

As detection technologies, JP-A-08-333398 discloses an immunoassay method making use of a polyclonal or monoclonal antibody which specifically recognizes cortisol (a kind of hormone) in urine; JP-A-2007-000009 discloses a method for easily and accurately detecting target nucleic acid molecules by using a degradative enzyme specific to double-stranded nucleic acids; JP-A-2006-133098 discloses a detection method of a protein, which detects the existence or non-existence of the protein by inserting an active electrode in a separation medium, through which a protein sample has been subjected to electrophoresis, to oxidize functional groups of the protein and measuring the resulting electric current; JP-A-2002-296274 discloses a method for detecting tumor cells and their precursors in an uterocervical smear preparation by concurrently staining and detecting at least two different molecular markers, which indicate disease-associated variations in gene expression, with an antibody or nucleic acid probe; and JP-A-2006-262775 discloses a detection method of microorganism cells, which is characterized by bringing a luminescence sensitizer into contact with a sample of microorganism cells to increase the intensity of fluorescence to be emitted by the microorganism cells.

Here, a description is made about "aptamer" which is relevant to the present invention. The term "aptamer" means a nucleic acid molecule or peptide having a function that allows the nucleic acid molecule or peptide to specifically bind to a specific substance such as a protein, an organic small molecule, a nucleic acid, a multimeric complex, cells, a cell tissue, metal ions, a microorganism, a virus or the like. An "aptamer" can bind to any object without limitation, and is allowed to bind to an object with high affinity and specificity. Its mass synthesis is easy, and its acting mechanism is simple. It can, therefore, be used widely in areas such as structural proteomics, target analysis, target validation, and drug developments.

For the above-described reasons, technological developments on various aptamers are under way in recent years. For example, JP-A-2006-320289 discloses an RNA aptamer, which binds specifically to fibrils derived from abnormal prion protein (mSAF) and is usable for the diagnosis, treatment or prevention of prion disease; and JP-A-2007-082543 discloses an RNA aptamer which inhibits Escherichia coli release factors.

WO 00/22167 discloses certain transcription factor significantly increasing the transcription rates from genes by nicking a single DNA strand in the vicinity of their DNA binding sides, thereby allowing RNA polymerizing to gain access to the transcribed DNA strand by a process of "threading".

From US 2003/0162211 A1, a method is known with which an activation profile of a biological sample is obtained, comprising disposing onto a solid support in a predetermined spatial arrangement, a subset of capture molecules able to interact with one or more activated transcription factor(s) present in the biological cell sample, containing their biological sample upon the solid support under conditions allowing their interaction, monitoring signals resulting from their interaction, and providing a cellular activation profile from the detected signals.

US 6,261,783 B1 teaches a method for detecting a target molecule in a test mixture suspected of containing said target molecule. The nucleic acid ligand capable of binding to the target molecule has a first sequence A and a second sequence B which are partially complementary sequences that form an imperfect intramolecular duplex, which unwinds upon the binding of the target to the nucleic acid ligand.

US 2006/0040276 A1 discloses an article suitable for use as a biosensor including a molecule of the formula X-R-Ch adhered to a surface of the article as part of a self-assembled monolayer. X is a functionality that adheres to the surface, R is a spacer moiety and Ch is a gelating agent.

EP 1 521 077 A1 describes a detecting unit for detecting interaction between substances including a pair of first opposed electrodes disposed opposite to each other so as to sandwich a reaction area providing a field for the interaction between the substances, and both wear one of electrodes forming second opposed electrodes disposed opposite to each other in a direction of an axis crossing an opposing axis of a first opposed electrode.

### SUMMARY OF THE INVENTION

The above-described substance detection methods all have both merits and demerits. For example, each of them involves one or more problems in that it is not suited for the detection of small molecules, it cannot detect if a sample is in a trace amount, and/or it requires a special step to avoid deactivation of a substance.

Accordingly, there is still a room for further developments.

An object of the present invention is, therefore, to provide a novel detecting nucleic acid strand construct which in turn can provide a substance detection method not known to date.

This object is solved by the nucleic acid strand of claim 1 and the method of claim 2.

The present inventors conducted extensive research with a view to developing a substance detection method making use of the property of an aptamer. As a result, the present inventors radically changed the idea of measuring a change in a probe or target itself that had been practiced in the conventional methods, and have found a novel method capable of detecting a target by contriving to make a substance, which is different from an aptamer or the target, change upon binding of the aptamer with the target and measuring that change.

In the present invention, there is thus provided a detecting nucleic acid strand construct thereinafter also referred to as "detecting nucleic acid strand" comprising: a first nucleic acid strand having a first base sequence region capable of functioning as an aptamer, and a second nucleic acid strand having a second base sequence region, which is complementary to the first base sequence region and forms a complementary strand with the first base sequence region, wherein the detecting nucleic acid strand is designed such that, when a predetermined substance interacts with the first base sequence region, the complementary strand of the first and second base sequence regions dissociates into single strands.

The second nucleic acid strand of the detecting nucleic acid strand is labeled with a fluorescent material.

According to the present invention, a fluorescent material is labeled on the second nucleic acid strand and a quencher is labeled on the first nucleic acid stand capable of quenching the fluorescent material while the complementary strand of the first and second base sequence regions exists.

In a second aspect of the present invention, there is also provided a method for detecting an interaction between a first base sequence region capable of functioning as an aptamer and a predetermined substance by using at least the above-described detecting nucleic acid strand.

In the above-described method, the first nucleic acid strand can be immobilized at an end thereof on a solid-phase surface.

A description will now be made about certain technical terms relating to the present invention. The term "aptamer" as used herein means a nucleic acid molecule or peptide having a function that allows the nucleic acid molecule or peptide to specifically bind to a specific substance such as a protein, an organic small molecule, a nucleic acid, a multimeric complex, cells, a cell tissue, metal ions, a microorganism, a virus or the like.

The term "probe" as used herein embraces all materials each of which can acquire a detection signal for the detection of a predetermined substance. Examples can include dielectrics, beads having desired weights, fluorescent materials, radioactive materials, intercalators, and the like.

The term "quencher" as used herein means a material, which is an excitation energy absorber and has a function to inhibit the emission of fluorescence from a nearby fluorescent material.

The term "intercalator" as used herein means a material that binds to a complementary strand site in a double-stranded nucleic acid to emit fluorescence or the like. Examples can include "POPO-1" (trade name, product of Molecular Probes, Inc.), "TOTO-3" (trade name, product of Invitrogen Corporation), "SYBR^{™} GREEN I"(product of Invitrogen Corporation), "PICOGREEN^{™}" (product of Molecular Probes, Inc.), and Hoechst 33258.

The use of the detecting nucleic acid strand according to the present invention makes it possible to perform simple and accurate detection of a predetermined substance irrespective of its size, amount, kind and the like. Further, the detecting nucleic acid strand according to the present invention can minimize a reduction in the activity of the predetermined substance, and therefore, does not require to conduct any additional step for the inhibition of the reduction in the activity. Furthermore, the detecting nucleic acid strand according to the present invention can detect a binding or interaction between substances with high accuracy.

The method according to the present invention to detect an interaction between substances can be applied to the analysis of a function of an aptamer, the analysis of a function of a predetermined substance after its binding to the aptamer or the screening of a substance, and is expected to make contributions to all areas such as the diagnosis of diseases, screening of compounds such as drugs, forensic medicine, comprehensive analysis of genetic information, function analysis of biosubstances, proteome analysis, analysis of *in vivo* interactions, hygienic control of foods and the like, and cleanup and restoration of environmental pollutions.

### BRIEF DESCRIPTION OF THE DRAWINGS

FIGS. 1A and 1B are schematic illustrations of a detecting nucleic acid strand representing background art for the understanding of the present invention and a method for detecting a substance with the detecting nucleic acid strand;
FIGS. 2A to 2C are schematic illustrations of a detecting nucleic acid strand representing background art for the understanding of the present invention and a method for detecting a substance with the detecting nucleic acid strand;
FIGS. 3A and 3B are schematic illustrations of a detecting nucleic acid strand according to the present invention and a method according to the second aspect of the present invention for detecting a substance with the detecting nucleic acid strand;
FIGS. 4A and 4B are schematic illustrations of a detecting nucleic acid strand representing background art for the understanding of the present invention and a method for detecting a substance with the detecting nucleic acid strand;
FIGS. 5A and 5B are schematic illustrations of a method representing background art for the understanding of the present invention for detecting an interaction between predetermined substances;
FIGS. 6A and 6B are schematic illustrations of a method representing background art for the understanding of the present invention for detecting an interaction between predetermined substances; and
FIGS. 7A to 7C are schematic illustrations of a method representing background art for the understanding of the present invention for the detection of a binding or interaction between substances as applied to the analysis of a function of an aptamer, the analysis of a function of a predetermined substance after its binding to the aptamer or the screening of a substance.

### DETAILED DESCRIPTION

With reference to the accompanying drawings, a description will hereinafter be made. It is, however, to be noted that the embodiments to be described hereinafter merely illustrate representative embodiments of the present invention by way of example and that the scope of the present invention shall not be narrowly interpreted by the following examples.

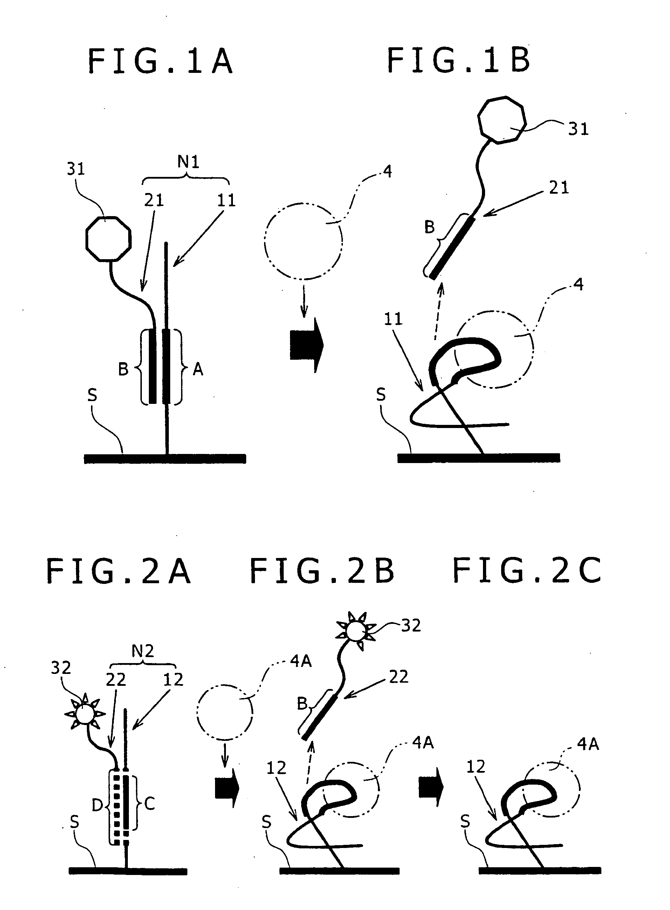

Referring firstly to FIGS. 1A and 1B, a description will be made of a detecting nucleic acid strand N1 and a method for detecting a predetermined substance 4 with the detecting nucleic acid strand N1.

Described roughly, the detecting nucleic acid strand N1 is provided at least with a first nucleic acid strand 11 and a second nucleic acid strand 21.

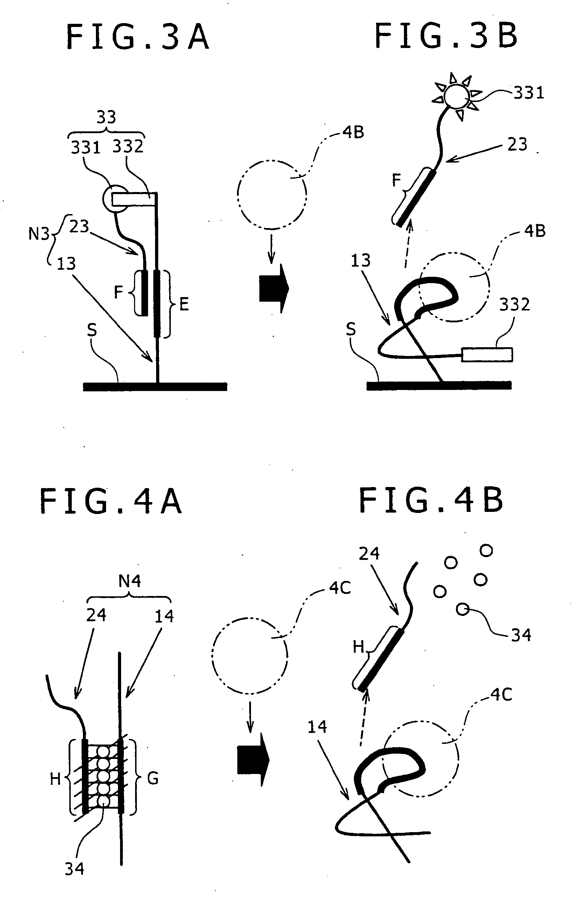

The first nucleic acid strand 11 has, as the first base sequence region, a base sequence region A which functions as an aptamer. In FIGS. 1A and 1B, the first nucleic acid strand 11 is immobilized at an end thereof on a solid-phase surface S (such as beads or the like; this applied equally hereinafter) although it is not limited to such an immobilized form. As will be described subsequently with reference to FIGS. 4A and 4B, it may be in a free form. Upon immobilizing the first nucleic acid strand 11 at the end thereof on the solid-phase surface S, no particular limitation is imposed on a method for its immobilization, and known methods are all usable. Illustrative are avidin-biotin binding and coupling reactions (for example, diazo-coupling reaction).

The second nucleic acid strand 21 has, as the second base sequence region, a base sequence region B complementary to the base sequence region A. In FIGS. 1A and 1B, a base sequence region complementary to the whole base sequence region A is shown as an example of the base sequence region B. The base sequence region B is, however, not limited to such a base sequence region, and as shown in FIGS. 2A to 2C, the base sequence region B may be complementary to a base sequence region longer than the base sequence region A. Further, the base sequence region B may be complementary to at least a part of the base sequence region A as depicted in FIGS. 3A and 3B.

In FIGS. 1A and 1B, the second nucleic acid strand 21 is modified at an opposite end thereof with a probe, that is, a dielectric 31. It is, however, unnecessary to modify the second nucleic acid strand 21 with the probe beforehand. For example, a modification, labeling or the like may be applied shortly before a detection. No particular limitation is imposed on the kind of the probe, although a substance capable of acquiring a physical or chemical detection signal is preferred. In addition to the dielectric 31, all known probes such as beads having known weights, fluorescent materials, radioactive materials and intercalators can be used.

It is the detecting nucleic acid strand N1 that the above-described first nucleic acid strand 11 and second nucleic acid strand 21 are in a double-stranded form.

In FIGS. 1A and 1B, numeral 4 designates a predetermined substance which specifically binds to the base sequence region A. No particular limitation is imposed on the predetermined substance 4 to be detectable, insofar as it is a substance that specifically binds or interacts to the base sequence region A which functions as an aptamer. Examples can include proteins, organic small molecules, nucleic acids, multimeric complexes, cells, cell tissues, metal ions, microorganisms, viruses, and the like.

In a place of reaction or interaction, the detecting nucleic acid strand N1 is ready for reaction or interaction (see FIG. 1A). When the predetermined substance 4 is charged there, the base sequence region A and the predetermined substance 4 bind to each other to result in dissociation of the second nucleic acid strand 21 as illustrated in FIG. 1B when the associativity between the predetermined substance 4 and the base sequence region A is more dominant over the associativity between the predetermined substance 4 and the base sequence region B. The strength of associativity can be adjusted by modifying the length of the base sequence region A or B and/or the GC content.

As the second nucleic acid strand 21 carries the dielectric 31 thereon for its modification, the dielectric constant on the solid-phase surface significantly changes when the second nucleic acid strand 21 dissociates. The predetermined substance 4 can, therefore, be detected by measuring a change in dielectric constant with a surface plasmon resonance sensor (SPR sensor) or the like.

When the probe carried on the second nucleic acid strand 21 for its modification is a bead having a desired weight, the predetermined substance 4 can also be detected by measuring a change in weight in accordance with the quartz crystal microbalance method (QCM method) or the like.

Different from commonly-employed probe nucleic acids, the detecting nucleic acid strand N1 is designed such that only the base sequence region A in the first nucleic acid strand 11 forming the double strand binds or interacts to the predetermined substance 4 and the other nucleic acid strand forming the double strand, i.e., the second nucleic acid strand 21 dissociates at the same time. It is, therefore, unnecessary to perform labeling or the like on the predetermined substance 4. Further, the detection of the predetermined substance 4 is performed by measuring a change in the dissociating second nucleic acid strand 21 rather than measuring a change in the predetermined substance 4 itself. It is, accordingly, possible to detect the predetermined substance 4 with high sensitivity even when the predetermined substance 4 is a small molecule or is in a trace amount.

With reference to FIGS. 2A to 2C, a description will next be made of a detecting nucleic acid strand N2 and a method for detecting a predetermined substance 4A with the detecting nucleic acid strand N2.

The detecting nucleic acid strand N2 is in a form that a base sequence region D as the second base sequence region in a second nucleic acid strain 22 complimentarily forms a double strand with a base sequence region which as the first base sequence region, is longer than the base sequence region C in a first nucleic acid strain 12. Further, a fluorescent material 32 as an illustrative probe is labeled to one end of the second nucleic acid strand 22. No particular limitation is imposed on the kind of the fluorescent material 32. For example, however, any known fluorescent material such as a florescent dye, e.g., Cy3 or Cy5 or a fluorescent protein, e.g., luciferase or GFP can be used.

Similar to the first embodiments shown in FIGS. 1A and 1B, the detecting nucleic acid strand N2 is ready for reaction or interaction in a place of reaction or interaction (see FIG. 2A). When a predetermined substance 4A is charged there, the base sequence region C and the predetermined substance 4A bind to each other to result in dissociation of the second nucleic acid strand 22 as illustrated in FIG. 2B.

The predetermined substance 4A can be detected by washing the place of reaction or interaction and then determining whether or not fluorescence is emitted by fluorescence excitation light of a predetermined wavelength. Described specifically, when the predetermined substance 4A which binds to or interacts with the base sequence region C exists, the second nucleic acid strands dissociates so that as illustrated in FIG. 2C, the second nucleic acid strand 22 is eliminated by subsequent washing and no fluorescence is emitted even when fluorescence excitation light of the predetermined wavelength is irradiated. When the predetermine substance 4A which binds to or interacts with the base sequence region C does not exist, the second nucleic acid strand 22 does not dissociate and the detecting nucleic acid strand N2 remains in the form of the double strand. As the detecting nucleic acid strand N2 is not eliminated by washing, fluorescence is emitted when fluorescence excitation light of the predetermined wavelength is irradiated.

Referring next to FIGS. 3A and 3B, a description will hereinafter be made of a detecting nucleic acid strand construct N3 according to the present invention and a method according to the present invention for detecting a predetermined substance 4B with the detecting nucleic acid strand construct N3.

The detecting nucleic acid strand construct N3 according to the present invention is in a form that a base sequence region F as the second base sequence region in a second nucleic acid strain 23 complimentarily forms a double strand with a part of a base sequence region E as the first base sequence region in a first nucleic acid strain 13. Employed is a fluorescent material 331 and a quencher 332 capable of quenching the florescent material 331. It is designed that the fluorescent material 331 is labeled to one end of the second nucleic acid strand 23 and the quencher 332 is labeled to one end of the first nucleic acid strand 13 to quench the fluorescent material 331 as long as the double strand is formed.

Similar to FIGS. 1A and 1B and FIGS. 2A to 2C, the detecting nucleic acid strand construct N3 is ready for reaction or interaction in a place of reaction or interaction with the fluorescent material 331 being in a quenched state (see FIG. 3A). When a predetermined substance 4B is charged there, a base sequence region E and the predetermined substance 4B bind to each other to dissociate a second nucleic acid strand 23 as illustrated in FIG. 3B.

Upon dissociation of the first nucleic acid strand 13 and the second nucleic acid strand 23 from each other, the fluorescent material 331 and the quencher 332 separate from each other so that the fluorescent material 331 becomes ready to emit fluorescence. Measurement of an emission of fluorescence by fluorescent excitation light of a predetermined wavelength, therefore, makes it possible to detect the predetermined substance 4B.

The use of a quencher according to the present invention can bring about a merit that a washing step such as that conducted in the second embodiments shown in FIGS. 2A to 2C is no longer required.

With reference to FIGS. 4A and 4B, a description will hereinafter be made of a detecting nucleic acid strand N4 and a method for detecting a predetermined substance 4C with the detecting nucleic acid strand N4.

The detecting nucleic acid strand N4 is not immobilized on any solid-phase surface, but is in a free state. As an illustrative probe, an intercalator 34 is used and is bound or adsorbed on a complementary strand of a base sequence region G as the first base sequence region in a first nucleic acid strand 14 and a base sequence region H as the second base sequence region in a second nucleic acid strand 24.

Similar to the embodiments shown in FIGS. 1A and 1B, FIGS. 2A to 2C and FIGS. 3A and 3B, the detecting nucleic acid strand N4 is ready for reaction or interaction in a place of reaction or interaction (see FIG. 4A). When a predetermined substance 4C is charged there, the base sequence region G and the predetermined substance 4C bind to each other to result in dissociation of the second nucleic acid strand 24 as illustrated in FIG. 4B.

Upon dissociation of the first nucleic acid strand 14 and the second nucleic acid strand 24 from each other, the intercalator 34 also dissociates. Measurement of a reduction in fluorescence emission under irradiation of fluorescence excitation light or the like of a predetermined wavelength, therefore, makes it possible to detect the predetermined substance 4C.

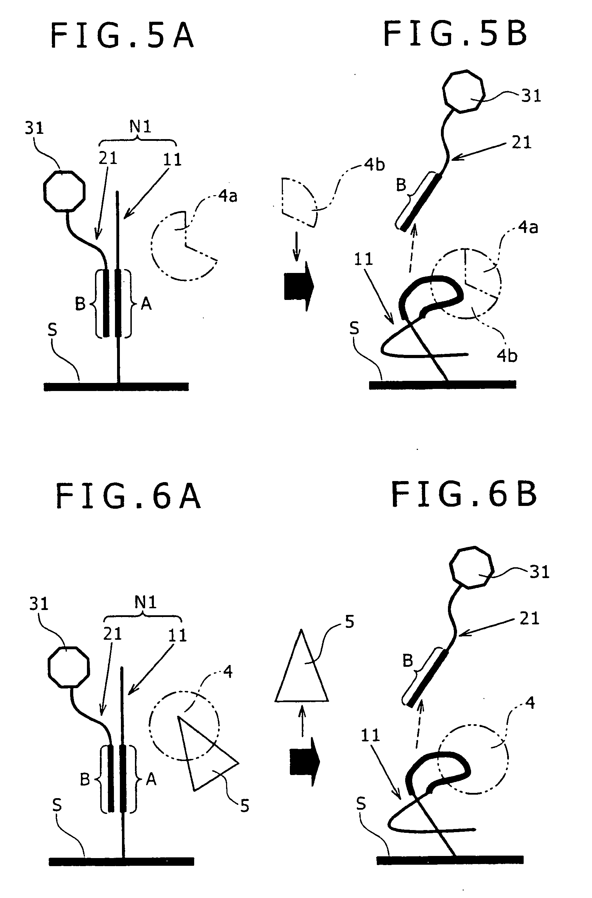

FIGS. 5A and 5B illustrate a method for detecting an interaction between predetermined substances 4a,4b. In this fifth figure, a description will be made using, as a detecting nucleic acid strand, the detecting nucleic acid strand N1 shown in FIGS. 1A and 1B. This is, however, not limited to the use of the detecting nucleic acid strand N1. For example, any one of the above-described detecting nucleic acid strands according to the first to fourth figures can also be used as desired.

The predetermined substance indicated at sign 4a in FIGS. 5A and 5B cannot bind to the base sequence region A as long as it exists as is. When it interacts with the other predetermined substance 4b, however, its properties are changed such that it can bind to the base sequence region A.

In a place of reaction or interaction, the detecting nucleic acid strand N1 and the predetermined substance 4a are ready for reaction or interaction (see FIG. 5A). When the predetermined substance 4b is charged there, an interaction takes place between the predetermined substance 4a and the predetermined substance 4b. The thus-interacted predetermined substance 4a,4b then binds to the base sequence region A to result in dissociation of the second nucleic acid strand 21.

As the second nucleic acid strand 21 carries the dielectric 31 thereon for its modification, the dielectric constant on the solid-phase surface significantly changes when the second nucleic acid strand 21 dissociates. The interaction between the predetermined substance 4a and the predetermined substance 4b can, therefore, be detected by measuring a change in dielectric constant, for example, with a surface plasmon resonance sensor (SPR sensor) or the like.

FIGS. 6A and 6B are schematic illustrations of a method for detecting an interaction between the predetermined substance 4 and a binding inhibition substance 5. In this sixth figure, a description will also be made using, as a detecting nucleic acid strand, the detecting nucleic acid strand N1 shown in FIGS. 1A and 1B. This is, however, not limited to the use of the detecting nucleic acid strand N1. For example, any one of the above-described detecting nucleic acid strands according to the first to fourth figures can also be used as desired.

In FIG. 6A, the binding inhibition substance 5 inhibits binding between the predetermined substance 4 and the base sequence region A. When the binding inhibition substance 5 dissociates from the predetermined substance 4 or the function of the binding inhibition substance 5 is lost under certain action, the predetermined substance 4 binds to the base sequence region A to result in dissociation of the second nucleic acid strand 21.

As the second nucleic acid strand 21 carries the dielectric 31 thereon for its modification, the dielectric constant on the solid-phase surface significantly changes when the second nucleic acid strand 21 dissociates. Dissociation of the binding inhibition substance 5 from the predetermined substance 4 or a functional failure of the binding inhibition substance 5 can, therefore, be detected by measuring a change in dielectric constant, for example, with a surface plasmon resonance sensor (SPR sensor) or the like.

The method according to the sixth figure can be applied to the screening of a function inhibitor such as the binding inhibition substance 5. When the predetermined substance 4 exists in a state that it is inhibited from binding to the detecting nucleic acid strand N1, specifically the base sequence region A by the binding inhibition substance 5 as shown in FIG. 6A and an unillustrated substance capable of acting as a function inhibitor for the binding inhibition substance 5 is then fed to inhibit the function of the binding inhibition substance 5, the predetermined substance binds to the base sequence region A. This binding results in dissociation of the second nucleic acid strand 21 so that the dielectric constant on the solid-phase surface significantly changes. As readily appreciated from the foregoing, the detecting nucleic acid strand N1 can be used in the screening of a function inhibitor for the binding inhibition substance 5.

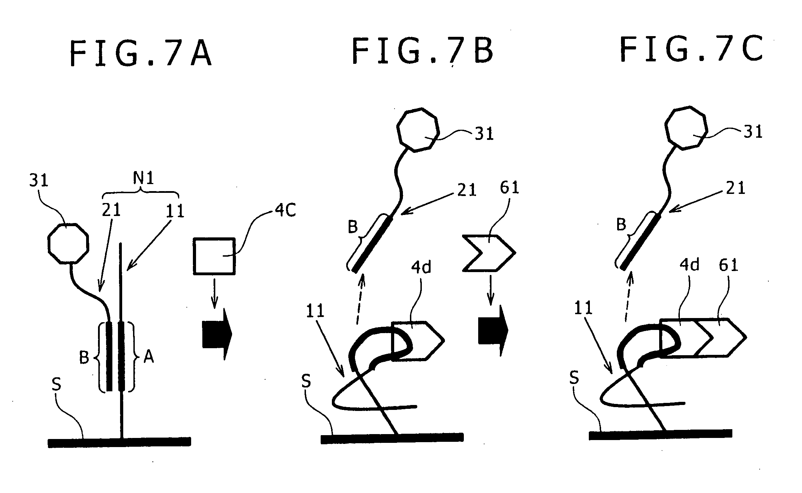

The above-described methods making use of the respective detecting nucleic acid strands can also be applied to the analysis of functions and the like to be described subsequently herein. One example of such applications will hereinafter be described with reference to FIGS. 7A to 7C.

FIGS. 7A to 7C illustrates a method for the detection of a binding or interaction between substances as applied to the analysis of a function of an aptamer, the analysis of a function of a predetermined substance after its binding to the aptamer or the screening of a substance. In this figure, a description will also be made using, as a detecting nucleic acid strand, the detecting nucleic acid strand N1 shown in FIGS. 1A and 1B. This is, however, not limited to the use of the detecting nucleic acid strand N1. For example, any one of the above-described detecting nucleic acid strands according to the first to fourth figures can also be used as desired.

As mentioned above, the detecting nucleic acid strand N1 is ready for reaction or interaction in a place of reaction or interaction (see FIG. 7A). When a predetermined substance 4c is charged there, the predetermined substance 4c and the base sequence region A bind to each other to result in dissociation of the second nucleic acid strand 21 as illustrated in FIG. 7B. The binding of the predetermined substance 4c to the base sequence region A, which functions as an aptamer, can firstly be confirmed by measuring a change in dielectric constant or the like at this time.

An aptamer is equipped with a function that its binds to various substances to affect their effects. When the predetermined substance 4c which normally does not undergo any interaction, for example, with a substance 61 becomes capable of interacting with the substance 61 subsequent to its binding to the aptamer (base sequence region A), this aptamer (base s sequence region A) is then appreciated to have a function to modify the predetermined substance 4c into a substance 4d which can interact with the substance 61.

When it is unknown what function the predetermined substance 4c would be provided with subsequent to its binding to the aptamer, the method according to this makes it possible to perform a functional analysis of the substance 4d obtained by the binding of the predetermined substance 4c to the aptamer (base sequence region A). If the predetermined substance c can be ascertained to have become capable of interacting with the substance 61 subsequent to its binding to the aptamer (base sequence region A) as illustrated in FIG. 7C, the substance 4d resulted from the binding of the predetermined substance 4c to the aptamer (base sequence region A) is appreciated to be equipped with such properties as permitting its interaction with the substance 61.

Further, the use of the principle of the above-described method also makes it possible to perform screening of the substance 61 available from the interaction of the predetermined substance 4c with the aptamer (base sequence region A).

In the above-described applications, the existence or non-existence of the interaction between the substance 61 and the substance 4c can be determined by a conventionally-known method. As the weight changes through the interaction between the substance 61 and the substance 4d, the interaction can be detected by the quartz crystal microbalance method (QCM method) or the like. The interaction can also be detected by a method that a labeling material, such as a fluorescent material, radioactive material or intercalator, is bound to or adsorbed on the substance 61 or substance 4d or by a method that as in the principle of PRET, the color or the like of fluorescence from the substance 61 or substance 4d itself is caused to change by the interaction between the substances.

## Claims

1. A detecting nucleic acid strand construct comprising:
a first nucleic acid strand having a first base sequence region capable of functioning as an aptamer, and
a second nucleic acid strand having a second base sequence region, which is complementary to said first base sequence region and forms a complementary strand with said first base sequence region, wherein
said second nucleic acid strand is labeled with a fluorescent material, and
said first nucleic acid strand is labeled with a quencher capable of quenching said fluorescent material while said complementary strand of said first and second base sequence regions exists;
wherein said detecting nucleic acid strand construct is designed such that, when a predetermined substance interacts with said first base sequence region by competing with the interaction between said first and said second sequence region, said complementary strand of said first and second base sequence regions dissociates into single strands.

2. A method for detecting an interaction between a first base sequence region capable of functioning as an aptamer and a predetermined substance by using at least a detecting nucleic acid strand according to claim 1.

3. The method according to claim 2, wherein said first nucleic acid strand is immobilized at an end thereof on a solid-phase surface.

## Patentansprüche

1. Detektionsnukleinsäurestrangkonstrukt, umfassend:
einen ersten Nukleinsäurestrang mit einer ersten Basensequenzregion, die dazu in der Lage ist, als Aptamer zu wirken, und
einen zweiten Nukleinsäurestrang mit einer zweiten Basensequenzregion, die komplementär zur ersten Basensequenzregion ist und einen komplementären Strang mit der ersten Basensequenzregion ausbildet, wobei
der zweite Nukleinsäurestrang mit einem Fluoreszenzmaterial markiert ist und
der erste Nukleinsäurestrang mit einem Quencher markiert ist, der dazu fähig ist, das Fluoreszenzmaterial zu quenchen, während der komplementäre Strang aus der ersten und zweiten Basensequenzregion existiert,
wobei das Detektionsnukleinsäurestrangkonstrukt so ausgebildet ist, dass, wenn eine vorbestimmte Substanz mit der ersten Basensequenzregion durch Kompetition mit der Wechselwirkung zwischen der ersten und zweiten Basensequenzregion interagiert, der komplementäre Strang aus den ersten und zweiten Basensequenzregionen in Einzelstränge dissoziiert.

2. Verfahren zum Nachweisen einer Wechselwirkung zwischen einer ersten Basensequenzregion, die dazu fähig ist, als Aptamer zu wirken, und einer vorbestimmten Substanz, indem mindestens ein Detektionsnukleinsäurestrangkonstrukt nach Anspruch 1 verwendet wird.

3. Verfahren nach Anspruch 2, wobei der erste Nukleinsäurestrang mit einem Ende davon an der Oberfläche einer festen Phase immobiolisiert ist.

## Revendications

1. Produit d'assemblage de brins d'acide nucléique de détection comprenant :
un premier brin d'acide nucléique présentant une première région de séquence de bases capable de fonctionner comme un aptamère, et
un second brin d'acide nucléique présentant une seconde région de séquence de bases qui est complémentaire de ladite première région de séquence de bases et forme un brin complémentaire avec ladite première région de séquence de bases, où
ledit second brin d'acide nucléique est marqué par un matériau fluorescent et
ledit premier brin d'acide nucléique est marqué par un désactivateur capable de désactiver ledit matériau fluorescent alors que ledit brin complémentaire desdites première et seconde régions de séquences de bases existe ;
où ledit produit d'assemblage de brins d'acide nucléique de détection est conçu de telle manière que, lorsqu'une substance prédéterminée interagit avec ladite première région de séquence de bases par compétition avec l'interaction entre ladite première et ladite seconde région de séquence, ledit brin complémentaire desdites première et seconde régions de séquences de bases se dissocie en brins simples.

2. Procédé de détection d'une interaction entre une première région de séquence de bases capable de fonctionner comme un aptamère et une substance prédéterminée en utilisant au moins un brin d'acide nucléique de détection selon la revendication 1.

3. Procédé selon la revendication 2, dans lequel ledit premier brin d'acide nucléique est immobilisé à une extrémité de celui-ci sur une surface de phase solide.
